# EUROPÄISCHE PATENTSCHRIFT

(11) **EP 3 847 151 B1**
(45) Veröffentlichungstag und Bekanntmachung des Hinweises auf die Patenterteilung: **09.11.2022**
(21) Anmeldenummer: 19761867.1
(22) Anmeldetag: 03.09.2019
(51) Int. Cl.: C07C 45/62, C07C 47/21

(54) **KONTINUIERLICHE HERSTELLUNG EINER OPTISCH AKTIVEN CARBONYLVERBINDUNG DURCH ASYMMETRISCHE HYDRIERUNG**
CONTINUOUS PROCESS FOR PREPARING AN OPTICALLY ACTIVE CARBONYL COMPOUNDS BY ASYMMETRIC HYDROGENATION
PROCÉDÉ CONTINU POUR PRÉPARER UN COMPOSÉ CARBONYLÉ OPTIQUEMENT ACTIF PAR HYDROGÉNATION ASYMÉTRIQUE

(30) Priorität: 05.09.2018 EP 18192756
(43) Veröffentlichungstag der Anmeldung: 14.07.2021
(73) Patentinhaber: BASF SE, 67056 Ludwigshafen am Rhein (DE)
(72) Erfinder: ZEHNER, Peter, 67273 Weisenheim am Berg (DE); BEY, Oliver, 67056 Ludwigshafen (DE)
(86) Internationale Anmeldenummer: PCT/EP2019/073456
(87) Internationale Veröffentlichungsnummer: WO 2020/048975

(56) Entgegenhaltungen:
- EP-A2- 0 753 501
- US-A1- 2018 057 437

## Beschreibung

Die vorliegende Erfindung betrifft ein Verfahren zur Herstellung optisch aktiver Carbonylverbindungen durch asymmetrische Hydrierung einer prochiralen α,β-ungesättigten Carbonylverbindung mit Wasserstoff in Gegenwart wenigstens eines homogenen Rhodium-Katalysators, der wenigstens einen chiralen Liganden aufweist. Speziell betrifft die vorliegende Erfindung ein Verfahren zur Herstellung von optisch aktivem Citronellal.

Viele optisch aktive Aldehyde und Ketone stellen wertvolle Zwischenstoffe zur Synthese höher veredelter chiraler Wert- und Wirkstoffe dar und sind ihrerseits oft begehrte Riech- und Aromastoffe.

Menthol stellt eine der wichtigsten Aromachemikalien dar, deren Hauptmenge nach wie vor aus natürlichen Quellen isoliert wird. Ein totalsynthetischer Zugang für das natürlich vorkommende Enantiomere L-Menthol bedient sich der asymmetrischen (enantioselektiven) Hydrierung von Geraniol oder Nerol zu optisch aktivem Citronellal. Das optisch aktive Citronellal kann dann mit Säure zu L-Isopulegol cyclisiert und zu L-Menthol hydriert werden. Es besteht ständiger Optimierungsbedarf bezüglich der Wirtschaftlichkeit des Verfahrens und insbesondere des einleitenden asymmetrischen Hydrierschritts.

Die DE 198 54 637 A1 beschreibt einen Reaktor zur kontinuierlichen Druchführung von Gas-Flüssig-, Flüssig-Flüssig- und Gas-Flüssig-Fest-Reaktionen, mit einer nach unten gerichteten Strahldüse und einem konzentrisch angeordneten Leitrohr.

Die EP-A 0 000 315 betrifft ein Verfahren zur Herstellung von optisch aktivem Citronellal durch Hydrierung von Geranial oder Neral in Gegenwart eines im Reaktionssystem gelösten Katalysatorkomplexes aus Rhodium und einem chiralen Phosphin.

Die DE 10 2004 049 631 beschreibt ein Verfahren zur Herstellung optisch aktiver Carbonylverbindungen durch asymmetrische Hydrierung α,β-ungesättigter Carbonylverbindungen in Gegenwart von im Reaktionssystem löslicher, optisch aktiver Übergangsmetall-Katalysatoren, die mindestens einen Kohlenmonoxid-Liganden aufweisen.

Die WO 2009/153123 beschreibt ein kontinuierliches Verfahren zur Hydrierung organischer Verbindungen in einem mehrphasigen System in Gegenwart eines homogenen oder heterogenen Katalysators, wobei man das Verfahren in zwei Stufen durchführt, wobei die erste Stufe in einem Schlaufenreaktor mit externem Wärmetauscher und die zweite Stufe in einem Blasensäulenreaktor mit begrenzter Rückvermischung durchgeführt wird.

Die vorliegende Erfindung betrifft ein Verfahren zur kontinuierlichen Herstellung einer optisch aktiven Carbonylverbindung durch asymmetrische Hydrierung einer prochiralen α,β-ungesättigten Carbonylverbindung mit Wasserstoff in Gegenwart eines homogenen Rhodium-Katalysators, der wenigstens einen chiralen Liganden aufweist, wobei man
ein die prochirale α,β-ungesättigte Carbonylverbindung enthaltendes flüssiges Reaktionsgemisch in einem ersten, rückvermischten Reaktor einer gas/flüssigzweiphasigen Hydrierung unterzieht, und
das flüssige Reaktionsgemisch dann in einem zweiten Reaktor weiter hydriert,
wobei man in dem ersten Reaktor eine Konzentration der prochiralen α,β-ungesättigten Carbonylverbindung von 3 bis 20 Gew.-% einstellt.

Es wurde gefunden, dass die Reaktionsgeschwindigkeit der Hydrierung der prochiralen α,β-ungesättigten Carbonylverbindung mit Wasserstoff in Gegenwart des homogenen Rhodium-Katalysators bei niedrigen Konzentrationen der α,β-ungesättigten Carbonylverbindung zunächst mit der Konzentration der α,β-ungesättigten Carbonylverbindung steil ansteigt, dann aber bei höheren Konzentrationen der α,β-ungesättigten Carbonylverbindung scharf abnimmt. Vermutlich kann die olefinische Doppelbindung der α,β-ungesättigten Carbonylverbindung bei hohen Konzentrationen der α,β-ungesättigten Carbonylverbindung mit dem Rhodiumatom des Katalysators interagieren und den Katalystor reversibel hemmen. Ein derartiges Verhalten wird auch als Edukt-Hemmung bezeichnet.

Beim erfindungsgemäßen Verfahren setzt man ein flüssiges Reaktionsgemisch in einem ersten, rückvermischten Reaktor um. Die Rückvermischung mit dem Hydrierprodukt führt zu einer Verdünnung der α,β-ungesättigten Carbonylverbindung und einer Minimierung der Edukt-Hemmung.

Die Reaktionsgeschwindigkeit kann ausgedrückt werden als die umgesetzte Menge an α,β-ungesättigterCarbonylverbindung pro Zeit, normiert auf die Stoffmenge der Rhodium-Atome. Sie zeigt bei Auftragung (unter den Druck- und TemperaturBedingungen im ersten Reaktor) gegen die Konzentration der prochiralen α,β-ungesättigten Carbonylverbindung ein Maximum Vₘₐₓ. Vorzugsweise stellt man in dem ersten Reaktor eine Konzentration der prochiralen α,β-ungesättigten Carbonylverbindung ein, bei der die Reaktionsgeschwindigkeit wenigstens das 0,8-fache von Vₘₐₓ beträgt.

Im ersten Reaktor stellt man eine Konzentration der prochiralen α,β-ungesättigten Carbonylverbindung in der Flüssigphase von 3 bis 20 Gew.-%, vorzugsweise 5 bis 20 Gew.-%, insbesondere 5 bis 15 Gew.-%, wie 7 bis 13 Gew.-%, 8 bis 12 Gew.-% oder 9 bis 11 Gew.-%, beispielsweise 10 Gew.-% ein. Da trotz der Rückvermischung in dem ersten Reaktor die Konzentration der prochiralen α,β-ungesättigten Carbonylverbindung in der Flüssigphase nicht vollständig homogen ist, gilt als Konzentration der prochiralen α,β-ungesättigten Carbonylverbindung deren Konzentration in der Flüssigphase am Ausgang des ersten Reaktors.

Um den Umsatz zu erhöhen, wird das flüssige Reaktionsgemisch anschließend in einem zweiten Reaktor weiter umgesetzt. Hohe Konzentrationen an nicht umgesetzter prochiralen α,β-ungesättigten Carbonylverbindung (im Folgenden auch als "Edukt" bezeichnet) im finalen Reaktionsgemisch führen auch zu hohen Restkonzentrationen an prochiraler α,β-ungesättigten Carbonylverbindung im Katalysatorrückstand, von dem die optisch aktive Carbonylverbindung z. B. durch Destillation abgetrennt wird. Der Katalysatorrückstand wird üblicherweise nach einer weiter unten beschriebenen Präformierung in die Hydrierreaktion zurückgeführt. Hohe Restkonzentrationen an prochiraler α,β-ungesättigten Carbonylverbindung beeinträchtigen die Präformierung. Es ist daher wünschenswert, den Umsatz in dem zweiten Reaktor weitgehend zu vervollständigen und die Konzentration an nicht umgesetzter prochiraler α,β-ungesättigter Carbonylverbindung zu minimieren.

Vorzugsweise setzt man in dem zweiten Reaktor das flüssige Reaktionsgemisch bis zu einer Konzentration der prochiralen α,β-ungesättigten Carbonylverbindung von weniger als 5 Gew.-%, insbesondere weniger als 3 Gew.-%, wie weniger als 2 Gew.-% oder weniger als 1 Gew.-% um.

Vorzugsweise beträgt das Verhältnis des Reaktionsvolumens des ersten Reaktors zum Reaktionsvolumen des zweiten Reaktors 1:1 bis 1:5, insbesondere 1:2 bis 1:4, beispielsweise etwa 1:3,5.

Das Verweilzeitverhalten in einem Reaktor lässt sich durch das Kaskadenmodell abbilden (sh. beispielsweise Baerns, Hofmann, Renken, "Chemische Reaktionstechnik"), welches einen realen Reaktor durch eine theoretische Anzahl N (sogenannte Kesselzahl) idealer, in Reihe geschalteter Rührkessel beschreibt. Dabei gilt, dass die Kessel volumengleich sind und das Reaktionsvolumen sich nicht ändert. Eine Rückvermischung zwischen den einzelnen Kesseln ist ausgeschlossen. Die Kesselzahl N kann zwischen 1 und unendlich liegen, darf aber nur ganze Werte annehmen. Die Extremzustände sind N = 1 (vollständige Durchmischung, idealer Rührkessel) bzw. N = ∞ (keine axiale Rückvermischung, ideales Strömungsrohr).

Im erfindungsgemäßen Verfahren weist der erste Reaktor vorzugsweise eine Kesselzahl im Bereich von 1 bis 3, insbesondere 1 oder 2 auf.

Vorzugsweise ist der erste Reaktor als Schlaufenreaktor ausgebildet. Beispiele für Schlaufenreaktoren sind Rohrreaktoren mit internen und externen Schlaufen. Solche Reaktoren sind beispielsweise in Ullmann's Encyclopädie näher beschrieben (Ullmann's Encyclopedia of Industrial Chemistry, Verlag Chemie, Electronic Release 2008, 7th Edition, Kapitel "Stirred Tank and Loop Reactors" und Kapitel "Bubble Columns"). Ein Schlaufenreaktor besteht üblicherweise aus einem vertikalen, vorzugsweise zylindrischen Rohrreaktor.

Das Verhältnis von Länge zu Durchmesser des Schlaufenreaktors beträgt üblicherweise 2:1 bis 100:1, bevorzugt 5:1 bis 100:1, besonders bevorzugt 5:1 bis 50:1, insbesondere bevorzugt 5:1 bis 30:1.

Die Zuführung des Edukts erfolgt an einer beliebigen Stelle des Rohrreaktors, vorzugsweise durch eine Strahldüse, insbesondere eine oberhalb des Flüssigkeitsstandes angeordnete Strahldüse. Der Begriff Strahldüse bezeichnet in üblicher Weise ein sich in Strömungsrichtung verjüngendes Rohr. Die Strahldüse bewirkt eine Durchmischung des ersten Reaktors.

Der volumenspezifische Leistungseintrag in den ersten Reaktor beträgt vorzugsweise 0,5 bis 5 kW/m³, beispielsweise 1,0 bis 4 kW/m³ oder 1,5 bis 3 kW/m³. Der volumenspezifische Leistungseintrag kann bestimmt werden als Produkt aus Differenzdruck über die Strahldüse und Volumenstrom durch die Düse.

Die Strahldüse kann als Einstoff- oder Zweistoffdüse ausgelegt werden. Bei der Einstoffdüse wird nur das flüssige Reaktionsgemisch eingedüst und der Wasserstoff an einer beliebig anderen Stelle, vorzugsweise jedoch im unteren Bereich des Reaktors.

Vorteilhaft bei dieser Ausgestaltung ist der einfache Aufbau einer solchen Einstoffdüse. Das Eindüsen des Wasserstoffs im unteren Bereich des Reaktors unterstützt vorteilhafterweise die Zirkulation des Reaktionsgemischs im Reaktor. Bei der Zweistoffdüse wird der Wasserstoff zusammen mit dem flüssigen Reaktionsgemisch zugeführt und dispergiert.

In einer bevorzugten Ausführungsform wird das Reaktionsgemisch über eine am Kopf des Reaktors befindliche Einstoff-Strahldüse nach unten dosiert. Wasserstoff sammelt sich im Gasraum oberhalb des Flüssigkeitsstandes im Reaktor. Der Strahl aus der Düse fällt durch den Gasraum und schlägt beim Eintritt in die Flüssigphase das Wasserstoffgas ein, das auf diese Weise zu Bläschen zerteilt und in der Flüssigphase dispergiert wird.

Der Schlaufenreaktor ist im Allgemeinen als Rohrreaktor mit externem Kreislauf (externe Schlaufen) gestaltet. Bei einem Schlaufenreaktor mit externem Kreislauf befindet sich in der Regel ein Abzug an beliebiger Stelle des Reaktors, vorzugsweise im unteren Bereich des Reaktors, über den das Reaktionsgemisch in einem äußeren Kreislauf mittels eines Förderorgans der Strahldüse wieder zugeführt wird. Das Förderorgan ist vorzugsweise eine Pumpe, weshalb der externe Kreislauf üblicherweise als Umpumpkreislauf bezeichnet wird.

Beispiele für Pumpen sind Kreiselpumpen oder Rotationskolbenpumpen, wie Drehkolbenpumpen, Drehschieberpumpen, Kreiskolbenpumpen oder Zahnradpumpen. Besonders bevorzugt werden Kreiselpumpen als Förderorgan verwendet.

Vorzugsweise ist der erste Reaktor als Schlaufenreaktor mit externem Kreislauf ausgebildet, wobei sich im externen Kreislauf ein Wärmeaustauscher befindet. Ein solcher Reaktor wird im Rahmen dieser Erfindung als Schlaufenreaktor mit externem Wärmetauscher bezeichnet.

Der Wärmetauscher ist beispielsweise ein Rohrbündelwärmetauscher, Doppelrohrwärmetauscher, Plattenwärmetauscher oder Spiralwärmetauscher. Bei Auslegungsdrücken für den Reaktor unter 100 bar wird bevorzugt ein Rohrbündelwärmetauscher verwendet, bei höheren Drücken wird bevorzugt ein oder mehrere hintereinander geschaltete Doppelrohrwärmetauscher verwendet.

Der Schlaufenreaktor mit externem Wärmetauscher wird dabei üblicherweise so betrieben, dass ein Teil des Reaktionsgemisches aus dem Reaktor durch den externen Umpumpkreislauf, in dem sich der externe Wärmetauscher befindet, gefördert wird, wobei das durch den Wärmetauscher geförderte Reaktionsgemisch gekühlt wird. Durch das externe Umpumpen wird das Reaktionsgemisch in der ersten Reaktionsstufe in der Regel stark durchmischt und umgewälzt, so dass die Verweilzeit in der ersten Stufe üblicherweise der eines vollständig rückvermischten Rührkessels (CSTR) entspricht.

Das Reaktionsgemisch wird schließlich mittels der Strahldüse wieder dem Reaktor zugeführt. Üblicherweise werden frische Edukte und Katalysatorlösung in den Umpumpkreislauf dosiert und zusammen mit dem bereits im Umpumpkreislauf befindlichen Strom als Reaktionsgemisch dem Reaktor zugeführt.

In einer bevorzugten Ausführungsform ist der Schlaufenreaktor so gestaltet, dass zusätzlich zum externen Kreislauf eine so genannte interne Schlaufenströmung ausgebildet wird. In einem Schlaufenreaktor mit interner Schlaufenströmung ist in der Regel im Inneren des Rohrreaktors ein konzentrisches, vorzugsweise zylindrisches Leitrohr angeordnet, das sich im Wesentlichen über die gesamte Länge des Rohrreaktors mit Ausnahmen der Reaktorenden erstreckt.

Das Leitrohr ist in der Regel als einfaches Rohr ausgestaltet. Das Verhältnis von Länge zu Durchmesser des Leitrohrs beträgt im Allgemeinen 5:1 bis 100:1, bevorzugt 5:1 bis 50:1.

Der Durchmesser des Leitrohrs ist geringer als der Durchmesser des Rohrreaktors. Das Verhältnis des Durchmessers des Leitrohrs zum Durchmesser des Rohrreaktors beträgt in der Regel 0,3:1 bis 0,9:1, bevorzugt 0,5:1 bis 0,7:1. Der Raum zwischen Leitrohr und der Reaktorinnenwand wird im Allgemeinen als Ringraum bezeichnet.

Die Strahldüse ist üblicherweise so angeordnet, dass der von der Strahldüse erzeugte Gas/Flüssigkeitsstrahl in das Leitrohr gerichtet ist. Die Strahldüse ist bevorzugt oberhalb des oberen Ende des Leitrohrs angeordnet. Die Düsenspitze befindet sich oberhalb des Flüssigkeitsstandes und taucht nicht in die Flüssigphase ein. Der mittels Strahldüse erzeugte Gas/Flüssigkeitsstrahl bewirkt eine abwärts gerichtete Strömung im Leitrohr (Abstromsäule), die nach Verlassen des Leitrohrs umgelenkt wird, so dass die Flüssigkeit im Ringraum zwischen Leitrohr und Reaktorinnenwand in Richtung der Strahldüse wieder aufströmt (Aufstromsäule). So entsteht in der Regel eine interne Schlaufenströmung. Das Verhältnis der Volumenströme von interner Schlaufenströmung zu extern umgepumpten Reaktionsgemisch beträgt vorzugsweise 2 bis 30:1, besonders bevorzugt 5 bis 20:1.

Aus dem ersten Reaktor wird zumindest ein Teil des Reaktionsgemisches dem zweiten Reaktor zugeführt. In dem zweiten Reaktor ist die Rückvermischung vorzugsweise begrenzt, so dass die Verweilzeitverteilung in diesem zweiten Reaktor der eines Rohrreaktors angenähert ist. Durch diese definierte Flüssigkeitsverweilzeit kann der Umsatz des Edukts nahezu vollständig erfolgen. Der zweite Reaktor weist vorzugsweise eine Kesselzahl von mehr als 4, insbesondere mehr als 5 oder mehr als 6 auf.

Der zweite Reaktor besteht üblicherweise aus einem vertikalen, vorzugsweise zylindrischen Rohrreaktor. Das Verhältnis von Länge zu Durchmesser des Reaktors beträgt üblicherweise 2:1 bis 100:1, bevorzugt 5:1 bis 50:1, besonders bevorzugt 7:1 bis 25:1.

In einem zum Austritt aus dem zweiten Reaktor gelegenen Abschnitt des zweiten Reaktors erfolgt die Hydrierung vorzugsweise flüssig-einphasig, d. h. es liegt im zum Austritt aus dem zweiten Reaktor gelegenen Abschnitt keine dispergierte Gasphase vor und die Hydrierung erfolgt ausschließlich mit dem in der Flüssigphase gelösten Wasserstoff. Da die Hydrierumsätze zum Austritt aus dem zweiten Reaktor hin gering sind, ist die Konzentration an gelöstem Wasserstoff ausreichend. Durch die Abwesenheit einer diskreten Gasphase zum Austritt aus dem zweiten Reaktor hin kann der Flüssigkeits-Holdup des zweiten Reaktors vergrößert und die Verweilzeit der Flüssigphase im zweiten Reaktor erhöht werden. Da die Hydrierung in der Flüssigphase erfolgt, wird der Reaktionsraum auf diese Weise optimal genutzt. Auf den flüssig-einphasig betriebenen Abschnitt des zweiten Reaktors entfallen vorzugsweise 30 bis 50 % des Gesamtvolumens des zweiten Reaktors.

Die Rückvermischung im zweiten Reaktor wird vorzugsweise durch Einbauten begrenzt. Durch den Einbau solcher Vorrichtungen werden in der Regel die Zirkulation und damit die Rückvermischung von Gas- und Flüssigkeit eingeschränkt.

Die Begrenzung der Rückvermischung im zweiten Reaktor kann durch den Einbau verschiedener Einbauten realisiert werden. In einer bevorzugten Ausführungsform erfolgt die Begrenzung der Rückvermischung durch den Einbau von mehreren, fest angeordneten Böden in den Rohrreaktor. Es entstehen zwischen den einzelnen Böden somit Einzelsegmente ("Kompartimente") mit definierten Reaktionsvolumina. Jedes der Einzelsegmente wirkt in der Regel dabei wie ein einzelner, rückvermischter Rührkesselreaktor. Mit zunehmender Anzahl von Einzelsegmenten hintereinander nähert sich die Verweilzeitverteilung einer derartigen Kaskade in der Regel der Verweilzeit eines Rohrreaktors an. Die Anzahl der so gebildeten Einzelsegmente beträgt vorzugsweise 2 bis 20, besonders bevorzugt 2 bis 10, insbesondere bevorzugt 3 bis 6. Die Böden sind dabei bevorzugt als flüssigkeitsdurchlässige Böden ausgeführt. Besonders bevorzugt sind die Böden als Lochbleche gestaltet.

Um für eine ausreichende Wasserstoff-Sättigung zu sorgen, dispergiert man vorzugsweise in einem zum Eintritt in den zweiten Reaktor hin gelegenen Abschnitt des zweiten Reaktors, z. B. im ersten Einzelsegment des zweiten Reaktors, Wasserstoffgas in dem flüssigen Reaktionsgemisch.

Der zum Eintritt in den zweiten Reaktor gelegene Abschnitt des zweiten Reaktors ist hierzu vorzugsweise als Schlaufenreaktor mit externem Kreislauf gestaltet. Das Dispergieren von Wasserstoff in den Abschnitt erfolgt vorzugsweise durch eine Strahldüse. Die Strahldüse kann als Einstoff- oder Zweistoffdüse ausgelegt werden. Es befindet sich ein Abzug an beliebiger Stelle des Abschnitts über den das Reaktionsgemisch in einem äußeren Kreislauf mittels eines Förderorgans der Strahldüse wieder zugeführt wird. Der Wasserstoff kann zusammen mit dem flüssigen Reaktionsgemisch zugeführt oder an einer beliebigen Stelle in den zum Eintritt in den zweiten Reaktor gelegenen Abschnitt dosiert werden.

Vorzugsweise umfasst der Schlaufenreaktor mit externem Kreislauf einen Wärmetauscher und wird so betrieben, dass das aus dem Reaktor durch den externen Umpumpkreislauf geführte Reaktionsgemisch durch den Wärmetauscher gekühlt wird.

In einer bevorzugten Ausführungsform wird das Reaktionsgemisch über eine am Kopf des zweiten Reaktors befindliche Einstoff-Strahldüse nach unten dosiert. Der Strahl aus der Düse fällt durch den Gasraum und schlägt beim Eintritt in die Flüssigphase das Gas ein, das auf diese Weise zu Bläschen zerteilt und in der Flüssigphase dispergiert wird.

Die Anzahl der Einzelsegmente des zweiten Reaktors beträgt bevorzugt 2 bis 10, besonders bevorzugt 2 bis 10, insbesondere bevorzugt 3 bis 6. Das Volumen des ersten Einzelsegments, in dem Wasserstoffgas in dem flüssigen Reaktionsgemisch dispergiert wird, beträgt bevorzugt 30 bis 70%, bezogen auf das Gesamtvolumen des zweiten Reaktors. Das zweite bis letzte Einzelsegment des zweiten Reaktors werden vorzugsweise flüssig-einphasig betrieben; auf das zweite bis letzte Einzelsegment des zweiten Reaktors entfallen dann vorzugsweise 30 bis 70 % des Gesamtvolumens des zweiten Reaktors.

Der erste Reaktor und der zweite Reaktor sind vorzugsweise als zwei räumlich voneinander getrennte Apparate angeordnet, die über Rohrleitungen miteinander verbunden sind.

In anderen Ausführungsformen Ausführungsform können beide Reaktoren in einem Apparat (Hydrierreaktor) angeordnet sein. In dieser bevorzugten Ausführungsform ist der Hydrierreaktor als langes, zylindrisches Rohr ausgeführt (hochzylindrische Bauform).

Eine prochirale α,β-ungesättigte Carbonylverbindung kann durch Additionsreaktion an die olefinische Doppelbindung ein Chiralitätszentrum ausbilden. Hierzu trägt die Doppelbindung vier verschiedene Substituenten. Die prochirale α,β-ungesättigte Carbonylverbindung ist vorzugsweise ausgewählt unter Verbindungen der allgemeinen 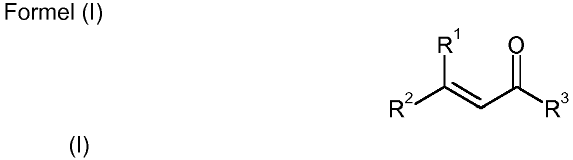 worin
- R¹, R²: voneinander verschieden sind und jeweils für einen unverzweigten, verzweigten oder cyclischen Kohlenwasserstoffrest mit 1 bis 25 Kohlenstoffatomen stehen, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen,
- R³: für Wasserstoff oder für einen unverzweigten, verzweigten oder cyclischen Kohlenwasserstoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen,
oder
- R³: gemeinsam mit einem der Reste R¹ oder R² auch eine 3 bis 25-gliedrige Alkylengruppe bedeuten kann worin 1, 2, 3 oder 4 nicht benachbarte CH₂-Gruppen durch O oder N-R^{5c} ersetzt sein können, wobei die Alkylengruppe gesättigt ist oder eine oder mehrere, nicht konjugierte ethylenische Doppelbindungen aufweist, und wobei die Alkylengruppe unsubstituiert ist oder einen oder mehrere gleiche oder verschiedene Substituenten trägt, die ausgewählt sind unter OR⁴, NR^{5a}R^{5b}, Halogen, C₁-C₄-Alkyl, C₆-C₁₀-Aryl und Hetaryl mit 5 bis 10 Ringatomen, wobei zwei Substituenten auch gemeinsam für eine 2 bis 10-gliedrige Alkylengruppe stehen können, wobei die 2- bis 10-gliedrige Alkylengruppe gesättigt ist oder eine oder mehrere, nicht konjugierte ethylenische Doppelbindungen aufweist, und wobei die 2- bis 10-gliedrige Alkylengruppe unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen;
wobei
- R⁴: für Wasserstoff, C₁-C₆-Alkyl, C₆-C₁₀-Aryl, C₆-C₁₄-Aryl-C₁-C₁₀-alkyl, oder C₁-C₁₀-Alkyl-C₆-C₁₄-aryl- steht;
- R^{5a}, R^{5b}: jeweils unabhängig voneinander Wasserstoff, C₁-C₆-Alkyl, C₆-C₁₀-Aryl, C₆-C₁₄-Aryl-C₁-C₁₀-alkyl oder C₁-C₁₀-Alkyl-C₆-C₁₄-aryl bedeuten oder
- R^{5a} und R^{5b}: gemeinsam auch eine Alkylenkette mit 2 bis 5 Kohlenstoffatomen, die durch N oder O unterbrochen sein kann, bedeuten können; und
- R^{5c}: für Wasserstoff, C₁-C₆-Alkyl, C₆-C₁₀-Aryl, C₆-C₁₄-Aryl-C₁-C₁₀-alkyl oder C₁-C₁₀-Alkyl-C₆-C₁₄-ary- steht.

In bevorzugten Ausführungsformen ist die prochirale α,β-ungesättigte Carbonylverbindung ausgewählt unter Verbindungen der allgemeinen Formeln (Ia) und (Ib) 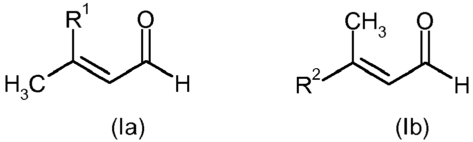 worin
- R¹, R²: jeweils für einen unverzweigten oder verzweigten Kohlenwasserstoffrest mit 2 bis 25 Kohlenstoffatomen steht, der gesättigt ist oder 1, 2, 3, 4 oder 5 nicht konjugierte ethylenische Doppelbindungen aufweist.

Eine besonders bevorzugte Ausführungsform betrifft ein Verfahren zur Herstellung von optisch aktivem Citronellal der Formel (III) 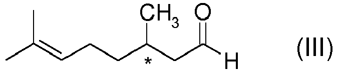 worin * das Asymmetriezentrum bezeichnet;
durch asymmetrische Hydrierung von Geranial der Formel (la-1) oder von Neral der Formel (lb-1) 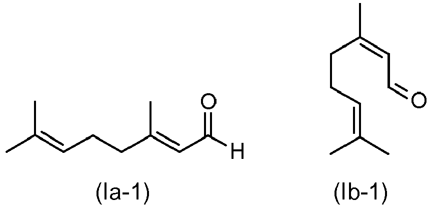
oder einer Neral und Geranial enthaltenden Mischung. Eine Neral und Geranial enthaltende Mischung ist unter der Bezeichnung Citral bekannt.

Man kann das so erhältliche optisch aktive Citronellal der Formel (III) einer Cyclisierung zu optisch aktivem Isopulegol unterziehen und das optisch aktive Isopulegol zu optisch aktivem Menthol hydrieren.

Durch das erfindungsgemäße Verfahren gelingt es, optisch aktive Carbonylverbindungen, insbesondere optisch aktive Aldehyde in hohen Ausbeuten und Enantiomerenüberschüssen bereitzustellen. Üblicherweise erhält man die gewünschten asymmetrisch hydrierten Verbindungen in einem Enantiomerenüberschuss von mindestens 80 % ee, oft mit einem Enantiomerenüberschuss von etwa 85 bis etwa 99 % ee. Dabei ist zu beachten, dass der maximal erzielbare Enantiomerenüberschuss von der Reinheit des eingesetzten Substrats, insbesondere im Hinblick auf die Isomerenreinheit der zu hydrierenden Doppelbindung, abhängen kann. Demzufolge eignen sich als Ausgangssubstanzen insbesondere solche, die ein Isomerenverhältnis von mindestens etwa 90:10, bevorzugt mindestens etwa 95:5 bezüglich der E/Z-Doppelbindungsisomere aufweisen.

Das erfindungsgemäße Herstellverfahren wird in Gegenwart eines im Reaktionsgemisch löslichen, optisch aktiven Rhodium-Katalysators durchgeführt, der mindestens einen optisch aktiven Liganden aufweist. Derartige Katalysatoren sind beispielsweise erhältlich durch Reaktion einer geeigneten, im Reaktionsgemisch löslichen Rhodium-Verbindung mit einem optisch aktiven Liganden, der mindestens ein Phosphor- und/oder Arsenatom aufweist.

Beispiele für erfindungsgemäß einsetzbare Rhodiumverbindungen sind: RhCl₃, Rh(OAc)₃, [Rh(cod)Cl]₂, Rh(CO)₂acac, [Rh(cod)OH]₂, [Rh(cod)OMe]₂, Rh₄(CO)₁₂, Rh₆(CO)₁₆, wobei "acac" für einen Acetylacetonat- und "cod" für einen Cyclooctadien-Liganden steht.

Die Katalysatorkonzentration im Reaktionsgemisch beträgt vorzugsweise 0,001 bis 1 mol-%, insbesondere 0,002 bis 0,5 mol-%, besonders bevorzugt 0,005 bis 0,2 mol-%, bezogen auf die Menge an prochiraler α,β-ungesättigter Carbonylverbindung im Reaktionsgemisch, gerechnet als im Katalysator enthaltene Rhodium-Atome.

Die genannten Rhodiumverbindungen werden mit einer weiteren Verbindung in Kontakt gebracht, die optisch aktiv, bevorzugt im wesentlichen enantiomerenrein ist (d.h. einen Enantiomerenüberschuss von mindestens etwa 99% aufweist) und mindestens ein Phosphor und/oder Arsenatom, bevorzugt mindestens ein Phosphoratom aufweist. Diese als chiraler Ligand zu bezeichnende Verbindung bildet im Reaktionsgemisch bzw. im Präformierungsgemisch mit der eingesetzten Rhodium-Verbindung den erfindungsgemäß einzusetzenden Rhodium-Katalysator.

Insbesondere bevorzugt sind solche chiralen Liganden die zwei Phosphoratome aufweisen und mit Rhodium Chelatkomplexe bilden.

Als chirale Liganden eignen sich im Rahmen der vorliegenden Erfindung solche Verbindungen, wie sie beispielsweise in: I. Ojima (Hrsg.), Catalytic Asymmetric Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Bevorzugte Liganden sind chirale zweizähnige Bisphosphin-Liganden, insbesondere solche der allgemeinen Formeln (IV) bis (VI) 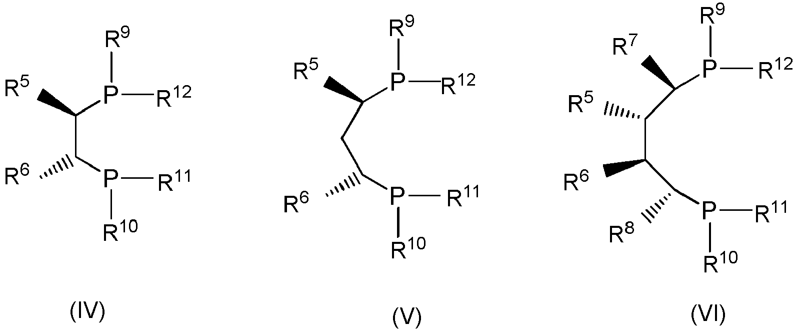 worin
R⁵, R⁶ jeweils unabhängig voneinander für einen unverzweigten, verzweigten oder cyclischen Kohlenwasserstoffrest mit 1 bis 20 Kohlenstoffatomen stehen, der gesättigt ist oder eine oder mehrere, in der Regel 1 bis etwa 4, nicht konjugierte, ethylenische Doppelbindungen aufweisen kann und der unsubstituiert ist oder einen oder mehrere, in der Regel 1 bis 4, gleiche oder verschiedene Substituenten trägt, die ausgewählt sind unter OR¹³, NR¹⁴R¹⁵, Halogen, C₆-C₁₀-Aryl und C₃-C₉-Hetaryl, oder
R⁵ und R⁶ gemeinsam eine 2 bis 10-gliedrige Alkylengruppe oder eine 3 bis 10-gliedrige Cycloalkylengruppe bedeuten kann, worin 1 , 2, 3 oder 4 nicht benachbarte CH-Gruppen durch O oder N-R¹³ ersetzt sein können, wobei die Alkylengruppe und die Cycloalkylengruppe gesättigt sind oder eine oder zwei, nicht konjugierte ethylenische Doppelbindungen aufweisen, und wobei die Alkylengruppe und die Cycloalkylengruppe unsubstituiert sind oder einen oder mehrere gleiche oder verschiedene Substituenten tragen, die ausgewählt sind unter C₁-C₄-Alkyl;
R⁷, R⁸ jeweils unabhängig voneinander Wasserstoff oder geradkettiges oder verzweigtes C₁-C₄-Alkyl bedeuten und
R9, R¹⁰, R¹¹, R¹² gleich oder verschieden sind und für C₆-C₁₀-Aryl stehen, das unsubstituiert ist oder einen oder mehrere Substituenten trägt, die ausgewählt sind unter C₁-C₆-Alkyl, C₃-C₆-Cycloalkyl, C₆-C₁₀-Aryl, C₁-C₆-Alkoxy und Amino;
R¹³, R¹⁴, R¹⁵ jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei R¹⁴ und R¹⁵ auch gemeinsam eine Alkylenkette mit 2 bis 5 Kohlenstoffatomen, die durch N oder O unterbrochen sein kann, bedeuten können.

Im Hinblick auf die Formeln (IV), (V) und (VI) haben die Variablen insbesondere die folgende Bedeutung:
R⁵, R⁶ stehen jeweils unabhängig voneinander für C₁-C₄-Alkyl oder
R⁵ und R⁶ stehen gemeinsam für einen C₃-C₅-Alkandiyl-Rest, C₃-C₇-Alkendiyl-Rest, C₅-C₇-Cycloalkandiyl-Rest oder einen C₅-C₇-Cycloalkendiyl-Rest, wobei die vier vorgenannten Reste unsubstituiert sind oder einen oder mehrere gleiche oder verschiedene Substituenten tragen, die ausgewählt sind unter C₁-C₄-Alkyl;
R⁷, R⁸ stehen jeweils unabhängig voneinander Wasserstoff oder C₁-C₄-Alkyl;
R⁹, R¹⁰, R¹¹, R¹² stehen jeweils für Phenyl.

Aufgrund ihrer leichten Verfügbarkeit besonders bevorzugte chirale, zweizähnige Bisphosphin-Liganden sind unter der Bezeichnung "Chiraphos" erhältliche Verbindungen der Formel:

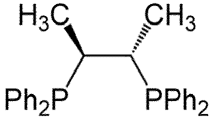

Die chiralen Liganden werden vorteilhaft in einer Menge von etwa 0,9 bis etwa 10 mol, bevorzugt etwa 1 bis etwa 4 mol pro mol eingesetzter Rhodium-Verbindung eingesetzt. Geeigneterweise wird der im Reaktionsgemisch lösliche, optisch aktive Rhodium-Katalysator durch Umsetzung einer achiralen Rhodiumverbindung und mit einem chiralen, zweizähnigen Bisphosphin-Liganden vor oder während der Hydrierung in-situ generiert. In diesem Zusammenhang bedeutet der Ausdruck "in-situ", dass der Katalysator direkt vor oder zu Beginn der Hydrierung generiert wird. Bevorzugt wird der Katalysator vor der Hydrierung generiert.

Es wurde gefunden, dass die Gegenwart einzähniger Liganden die Aktivität des Katalysators erhöhen kann. In einer bevorzugten Ausführungsform des erfindungsgemäßen Verfahrens werden Verbindungen der Formel (II) 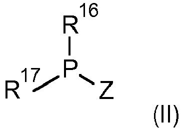 eingesetzt, worin Z in Formel (II) für eine Gruppe CHR¹⁸R¹⁹ steht und worin die Variablen R¹⁶, R¹⁷, R¹⁸, R¹⁹ unabhängig voneinander und insbesondere gemeinsam die folgenden Bedeutungen aufweisen:
- R¹⁶, R¹⁷:: sind gleich oder verschieden und stehen für Phenyl, das unsubstituiert ist oder 1, 2 oder 3 Substituenten trägt, die ausgewählt sind unter Methyl und Methoxy, wobei R¹⁶ und R¹⁷ jeweils insbesondere für unsubstituiertes Phenyl stehen;
- R¹⁸: steht für C₁- bis C₄-Alkyl, insbesondere für Methyl;
- R¹⁹: steht für C₁- bis C₄-Alkyl, das eine Gruppe P(=O)R^{19a}R^{19b} trägt, wobei und insbesondere eine Gruppe CH₂-P(=O)R^{19a}R^{19b} oder CH(CH₃)-P(=O)R^{19a}R^{19b} bedeutet;
wobei
- R^{19a}, R^{19b}:: gleich oder verschieden sind und für Phenyl stehen, das unsubstituiert ist oder 1, 2 oder 3 Substituenten trägt, die ausgewählt sind unter Methyl und Methoxy, wobei besonders bevorzugt R^{19a} und R^{19b} jeweils für unsubstituiertes Phenyl stehen.

In dieser bevorzugten Ausführungsform des erfindungsgemäßen Verfahrens wird besonders bevorzugt eine Verbindung der Formel (II) eingesetzt, worin
- R¹⁶, R¹⁷:: für unsubstituiertes Phenyl stehen;
- R¹⁸: für Methyl steht;
- R¹⁹: eine Gruppe CH(CH₃)-P(=O)R^{19a}R^{19b} bedeutet, worin R^{19a} und R^{19b} jeweils für unsubstituiertes Phenyl stehen.

Hierbei handelt es sich um die Verbindung (2-(Diphenylphosphoryl)-1-methylpropyl)-diphenylphosphan (Chiraphosmonooxid), einschließlich des (R,R)-Enantiomers (= (*R,R*)-Chiraphosmonooxid) und des (S,S)-Enantiomers (= (*S,S*)-Chiraphosmonooxid) sowie Gemischen aus (*R,R*)-Chiraphosmonooxid und (*S*,*S*)-Chiraphosmonooxid.

Falls die Reste R¹⁸ und R¹⁹ in der allgemeinen Formel (II) unterschiedliche Bedeutung haben, kann das Kohlenstoffatom, welches die Reste R¹⁸ und R¹⁹ trägt, eine (R)- oder (S)-Konfiguration aufweisen. Diese Verbindungen der allgemeinen Formel (II) können als reine (R)- oder reine (S)-Stereoisomere oder als Mischungen davon vorliegen. In der Regel wird man in diesen Fällen die reinen (R)- und (S)-Stereoisomere einsetzen, wobei auch etwaige Stereoisomerengemische für den Einsatz in dem vorliegenden Verfahren geeignet sind.

Unter einem reinen Stereoisomeren versteht man hier und im Folgenden chirale Substanzen, die bezüglich des gewünschten Stereoisomeren einen Enantiomerenüberschuss (ee) von wenigstens 80%ee, insbesondere wenigstens 90%ee und speziell wenigstens 95%ee aufweisen.

Insbesondere wird als chiraler Ligand Chiraphos verwendet und als einzähnig bindende Verbindung (2-(Diphenylphosphoryl)-1 -methylpropyl)-diphenylphosphan (Chiraphosmonooxid). Beispielsweise wird als chiraler Ligand R-Chiraphos verwendet und als einzähnig bindende Verbindung (*R,R*)-Chiraphosmonooxid und/oder (*S,S*)-Chiraphosmonooxid. Alternativ wird als chiraler Ligand S-Chiraphos verwendet und als einzähnig bindende Verbindung (*R,R*)-Chiraphosmonooxid und/oder (*S,S*)-Chiraphosmonooxid.

Die Verbindung der Formel (II) wird erfindungsgemäß in der Regel in einer Menge von 0,01 bis 1 Mol, bevorzugt 0,02 bis 0,8 Mol, besonders bevorzugt 0,03 bis 0,7 Mol und insbesondere in einer Menge von 0,04 bis 0,6 Mol pro Mol Rhodium einsetzt.

Weitere Ausführungsformen des Rhodium-Katalysators und des einzähnigen Liganden sind in US 2018/0057437 A1, WO 2006/040096 A1 und WO 2008/132057 A1 beschrieben.

Der zur Hydrierung verwendete Wasserstoff wird im Allgemeinen im größeren stöchiometrischen Überschuss vom 1- bis 10-fachen, bevorzugt vom 1,1- bis 5-fachen der stöchiometrisch notwendigen Mengen verwendet. Er kann als Kreisgas in die Reaktion zurückgeführt werden. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden. Bevorzugt wird jedoch reiner Wasserstoff bzw. im Wesentlichen reiner Wasserstoff in das Verfahren eingesetzt.

Die erfindungsgemäße asymmetrische Hydrierung nimmt man vorteilhaft bei einem Druck von etwa 2 bis etwa 200 bar, insbesondere von etwa 10 bis etwa 100 bar, speziell bei etwa 60 bis etwa 100 bar und einer Temperatur von in der Regel etwa 0°C bis etwa 100°C, bevorzugt etwa 0°C bis etwa 30°C, insbesondere bei etwa 10°C bis etwa 30°C vor.

Die Wahl des zur Durchführung der erfindungsgemäßen asymmetrischen Hydrierung einzusetzenden Lösemittels ist von geringerer Bedeutung. Geeignete unter Reaktionsbedingungen inerte Lösungsmittel bzw. Lösungsmedium sind beispielsweise Ether, Tetrahydrofuran, Methyltetrahydrofuran, Toluol, Xylole, Chlorbenzol, Octadecanol, Biphenylether, Texanol, Marlotherm, Oxoöl 9N (Hydroformylierungsprodukte aus isomeren Octenen, BASF SE), Citronellal und dergleichen. Als Lösungsmedium können auch das Hydrierprodukt oder bei der Umsetzung eventuell anfallende hochsiedende Nebenprodukte dienen. Mit besonderem Vorteil führt man die asymmetrische Hydrierung im gleichen Lösemittel durch wie eine gegebenenfalls zuvor durchgeführte Präformierung.

In einer bevorzugten Ausführungsform des erfindungsgenäßen Verfahrens wird der Katalysator vor der Hydrierung mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Dies bedeutet, dass man den eingesetzten, im Reaktionsgemisch löslichen, d.h. homogenen Rhodium-Katalysator entweder vor der asymmetrischen Hydrierung mit einem Gasgemisch vorbehandelt, das Kohlenmonoxid und Wasserstoff enthält (d.h. eine sogenannte Präformierung durchführt) oder die asymmetrische Hydrierung in Gegenwart von dem Reaktionsgemisch zusätzlich zugeführtem Kohlenmonoxid durchführt oder eine Präformierung durchführt und anschließend die asymmetrische Hydrierung in Gegenwart von dem Reaktionsgemisch zusätzlich zugeführtem Kohlenmonoxid durchführt.

Die in dieser bevorzugten Ausführungsform eingesetzten, im Reaktionsgemisch löslichen Rhodium-Katalysatoren können daher zumindest in einer im Katalysezyklus durchlaufenen Form oder in einer dem eigentlichen Katalysezyklus vorgeschalteten Vorform mindestens einen CO-Liganden aufweisen, wobei es unerheblich ist, ob diese mindestens einen CO-Liganden aufweisende Katalysatorform die tatsächliche katalytisch aktive Katalysatorform darstellt. Um die gegebenenfalls CO-Liganden aufweisende Katalysatorformen zu stabilisieren, kann es vorteilhaft sein dem Reaktionsgemisch während der Hydrierung zusätzlich Kohlenmonoxid zuzuführen.

In dieser bevorzugten Ausführungsform wird die genannte Vorbehandlung der Katalysator-Vorstufe mit einem Gasgemisch umfassend 20 bis 90 Vol.-% Kohlenmonoxid, 10 bis 80 Vol-% Wasserstoff und 0 bis 5 Vol.-% weiterer Gase, wobei sich die genannten Volumenanteile zu 100 Vol.-% ergänzen, bei einem Druck von 5 bis 100 bar durchführt. Zusätzlich wird von dem so erhaltenen Katalysator vor Einsatz in der asymmetrischen Hydrierung überschüssiges Kohlenmonoxid abgetrennt. Unter dem Begriff überschüssiges Kohlenmonoxid ist dabei solches Kohlenmonoxid zu verstehen, das in dem erhaltenen Reaktionsgemisch in gasförmiger oder gelöster Form enthalten ist und nicht an den Rhodium-Katalysator bzw. dessen Vorstufe gebunden ist. Demgemäß entfernt man das überschüssige, nicht an den Katalysator gebundene Kohlenmonoxid zumindest weitgehend, d.h. in einem Ausmaß, dass sich eventuelle Restmengen gelösten Kohlenmonoxids in der folgenden Hydrierung nicht störend bemerkbar machen. Dies ist üblicherweise gewährleistet, wenn etwa 90%, bevorzugt etwa 95% oder mehr des zur Präformierung eingesetzten Kohlenmonoxids abgetrennt werden. Bevorzugt entfernt man das überschüssige Kohlenmonoxid vollständig von dem durch Präformierung erhaltenen Katalysator.

Die Abtrennung des überschüssigen Kohlenmonoxids vom erhaltenen Katalysator bzw. vom den Katalysator enthaltenden Reaktionsgemisch kann auf verschiedenen Wegen erfolgen. Bevorzugt entspannt man den Katalysator bzw. das durch Präformierung erhaltene, den Katalysator enthaltende Gemisch auf einen Druck von bis zu etwa 5 bar (absolut), vorzugsweise, speziell, bei Durchführung der Präformierung im Druckbereich von 5 bis 10 bar, auf einen Druck von weniger als 5 bar (absolut), bevorzugt auf einen Druck im Bereich von etwa 1 bar bis etwa 5 bar, vorzugsweise 1 bis weniger als 5 bar, besonders bevorzugt auf einen Druck im Bereich von 1 bis 3 bar, ganz besonders bevorzugt auf einen Druck im Bereich von etwa 1 bis etwa 2 bar, insbesondere bevorzugt auf Normaldruck, so dass gasförmiges, nicht gebundenes Kohlenmonoxid aus dem Produkt der Präformierung entweicht. Die vorstehend genannte Entspannung des präformierten Katalysators kann beispielsweise unter Einsatz eines Hochdruckabscheiders, wie er dem Fachmann an sich bekannt ist, erfolgen. Derartige Abscheider, bei denen die Flüssigkeit in der kontinuierlichen Phase ist, sind beispielsweise beschrieben in: Perry's Chemical Engineers' Handbook, 1997, 7. Aufl., McGraw-Hill, S. 14.95 und 14.96; die Verhinderung eines möglichen Tropfenmitrisses ist auf den Seiten 14.87 bis 14.90 beschrieben. Die Entspannung des präformierten Katalysators kann einstufig oder zweistufig bis zum Erreichen des gewünschten Druckes im Bereich von 1 bar bis etwa 5 bar erfolgen, wobei die Temperatur üblicherweise auf 10 bis 40°C sinkt. Alternativ kann die Abtrennung überschüssigen Kohlenmonoxids durch sogenanntes Strippen des Katalysators bzw. des den Katalysator enthaltenden Gemischs mit einem Gas, vorteilhaft mit einem unter den Reaktionsbedingungen inerten Gas, erreicht werden. Unter dem Begriff Strippen versteht der Fachmann dabei das Einleiten eines Gases in den Katalysator bzw. das den Katalysator enthaltende Reaktionsgemisch wie beispielsweise in W. R. A. Vauck, H. A. Müller, Grundoperationen chemischer Verfahrenstechnik, Deutscher Verlag für Grundstoffchemie Leipzig, Stuttgart, 10 Auflage, 1984, Seite 800, beschrieben. Als geeignete inerte Gase seien hierfür beispielhaft genannt: Wasserstoff, Helium, Neon, Argon, Xenon, Stickstoff und/oder CO2, bevorzugt Wasserstoff, Stickstoff, Argon.

Bevorzugt wird die asymmetrische Hydrierung anschließend mit Wasserstoff durchführt, der einen Kohlenmonoxidgehalt im Bereich von 50 bis 3000 ppm, insbesondere im Bereich von 100 bis 2000 ppm, speziell im Bereich von 200 bis 1000 ppm und ganz speziell im Bereich von 400 bis 800 ppm aufweist.

Führt man eine Präformierung des Rhodium-Katalysators durch, löst man üblicherweise die gewählte Rhodiumverbindung und den gewählten chiralen Liganden in einem geeigneten, unter den Reaktionsbedingungen inerten Lösungsmittel bzw. Lösungsmedium wie beispielsweise Ether, Tetrahydrofuran, Methyltetrahydrofuran, Toluol, Xylole, Chlorbenzol, Octadecanol, Biphenylether, Texanol, Marlotherm, Oxoöl 9N (Hydroformylierungsprodukte aus isomeren Octenen, BASF Aktiengesellschaft), Citronellal und dergleichen. Als Lösungsmedium können auch das Hydrierprodukt oder bei der Umsetzung eventuell anfallende hochsiedende Nebenprodukte dienen. Der resultierenden Lösung wird, vorteilhafterweise in einem geeigneten Druckreaktor bzw. Autoklaven, bei einem Druck von üblicherweise etwa 5 bis etwa 350 bar, bevorzugt von etwa 20 bis etwa 200 bar und besonders bevorzugt von etwa 50 bis etwa 100 bar ein Gasgemisch aufgepresst, das Wasserstoff und Kohlenmonoxid enthält. Bevorzugt setzt man zur Präformierung ein Gasgemisch ein, das etwa
30 bis 99 Vol.-% Wasserstoff,
1 bis 70 Vol.-% Kohlenmonoxid und
0 bis 5 Vol.-% weiterer Gase enthält, wobei sich die Angaben in Vol.-% zu 100 Vol.-% addieren.

Ein zur Präformierung insbesondere bevorzugtes Gasgemisch ist sogenanntes Synthesegas, das üblicherweise zu etwa 35 bis 55 Vol-% aus Kohlenmonoxid neben Wasserstoff und Spuren weiterer Gase besteht.

Die Präformierung des Katalysators wird üblicherweise bei Temperaturen von etwa 25°C bis etwa 100°C, bevorzugt bei etwa 40°C bis etwa 80°C durchgeführt. Die Präformierung ist üblicherweise nach etwa 1 h bis etwa 24 h, oft nach etwa 1 bis etwa 12 h abgeschlossen. Im Anschluss an die optional durchzuführende Präformierung führt man erfindungsgemäß die asymmetrische Hydrierung des gewählten Substrats durch. Nach vorangegangener Präformierung lässt sich das gewählte Substrat in der Regel mit gutem Erfolg mit oder ohne Zuführung von zusätzlichem Kohlenmonoxid durchführen. Wird keine Präformierung durchgeführt, kann die erfindungsgemäße asymmetrische Hydrierung entweder in Gegenwart von dem Reaktionssystem zugeführtem Kohlenmonoxid oder ohne die Zuführung von Kohlenmonoxid durchgeführt werden. Vorteilhafterweise führt man eine Präformierung wie beschrieben durch und setzt dem Reaktionsgemisch während der asymmetrischen Hydrierung zusätzliches Kohlenmonoxid zu.

Falls dem Reaktionssystem Kohlenmonoxid zugeführt wird, kann die Zuführung auf verschiedene Weise vorgenommen werden: So kann das Kohlenmonoxid beispielsweise dem zur asymmetrischen Hydrierung eingesetzten Wasserstoff beigemischt werden oder auch direkt gasförmig in die Reaktionslösung eindosiert werden. Bevorzugt wird das Kohlenmonoxid dem zur asymmetrischen Hydrierung eingesetzten Wasserstoff beigemischt.

Das Reaktionsprodukt lässt sich durch dem Fachmann an sich bekannte Verfahren, wie z.B. durch Destillation und/oder Entspannungsverdampfung, aus dem Reaktionsgemisch entfernen und der zurückbleibende Katalysator, gegebenenfalls nach abermaliger Präformierung, im Rahmen weiterer Umsetzungen nutzen. Im Rahmen der bevorzugten Ausführungsform verzichtet man vorteilhaft auf den Zusatz von Lösemitteln und führt die genannten Umsetzungen im umzusetzenden Substrat bzw. dem Produkt und gegebenenfalls in hochsiedenden Nebenprodukten als Lösemedium durch. Insbesondere bevorzugt ist die kontinuierliche Reaktionsführung unter Wiederverwendung bzw. Rückführung des erfindungsgemäß stabilisierten homogenen Katalysators.

Die Erfindung wird durch die beigefügten Figuren und das nachfolgende Beispiel näher veranschaulicht.
Fig. 1 zeigt eine Auftragung der Reaktionsgeschwindigkeit (relativ zur maximalen Reaktionsgeschwindigkeit) der Hydrierung von Citral in Gegenwart eines Chiraphos-Rhodiumkatalysators in Abhängigkeit von der Citral-Konzentration.
Fig. 2 zeigt schematisch eine zur Durchführung des erfindungsgemäßen Verfahrens geeignete Anlage.
Fig. 3 zeigt schematisch eine zur Durchführung des erfindungsgemäßen Verfahrens geeignete Anlage.

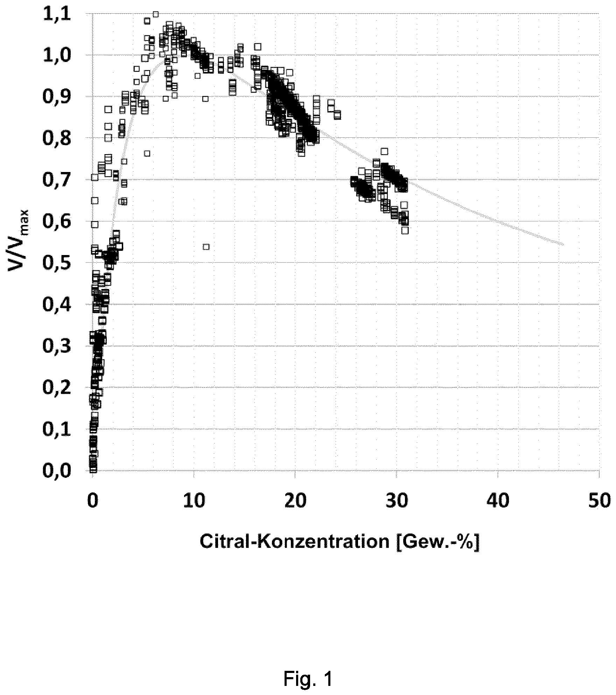

Die in Fig. 1 dargestellten Daten wurden in einem Strahlschlaufenreaktor gewonnen, in dem durch unterschiedliche Katalysatorbelastungen unterschiedliche Umsätze erzielt wurden. Dabei wurde die Differenz aus ein- und austretender Citral-Menge auf die im Reaktor voliegende Masse an Rhodium bezogen und über der Citral-Konzentration im Austrag aus dem Reaktor aufgetragen. Diese so ermittelte Reaktionsgeschwindigkeit wurde mit der maximalen Reaktionsgeschwindigkeit normiert.

Anhand von Fig. 1 ist erkennbar, dass die Reaktionsgeschwindigkeit der Citral-Hydrierung niedrigen Citral-Konzentrationen mit der Citral-Konzentration hyperbolisch ansteigt, dann aber bei höheren Citral-Konzentrationen scharf abnimmt. Das Maximum Vₘₐₓ der Reaktionsgeschwindigkeit wird bei einer Citral-Konzentration von etwa 9 Gew.-% erreicht. Reaktionsgeschwindigkeiten, die gleich dem 0,8-fachen von Vₘₐₓ oder höher sind, werden im Konzentrationsbereich von etwa 5 bis 20 Gew.-% Citral erreicht.

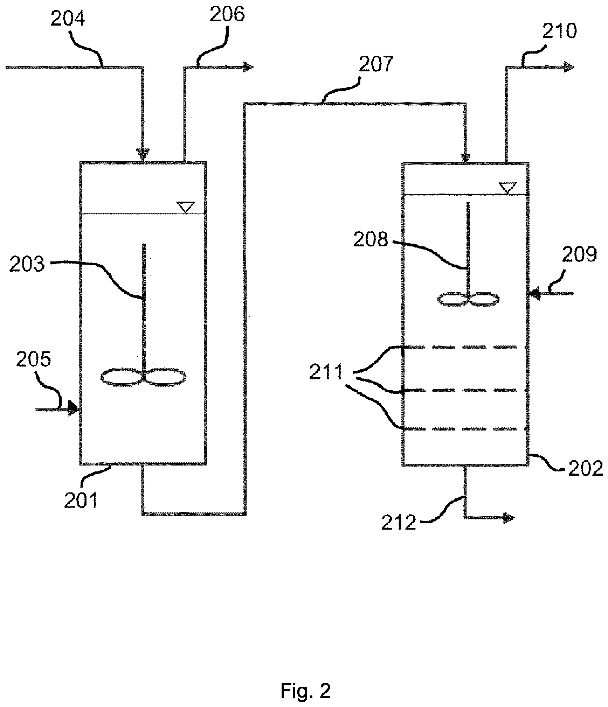

Gemäß Fig. 2 umfasst eine zur Durchführung des erfindungsgemäßen Verfahrens geeignete Anlage einen ersten Hydrierreaktor 201 und einen zweiten Hydrierreaktor 202.

Der Hydrierreaktor 201 umfasst einen Rührer 203. Dem Hydrierreaktor 201 wird eine ein zu hydrierendes Edukt umfassende Flüssigkeit über Leitung 204 zugeführt. Wasserstoffgas wird dem Hydrierreaktor 201 über Leitung 205 zugeführt. Das Abgas des Hydrierreaktors 201 wird über Leitung 206 aus dem Hydrierreaktor 1 ausgeführt.

Der am Sumpf des Hydrierreaktors 201 ausgeführte Austragsstrom 207 wird dem Hydrierreaktor 202 zugeführt. Der Hydrierreaktor 202 umfasst einen Rührer 208. Wasserstoffgas wird dem Hydrierreaktor 202 über Leitung 209 zugeführt. Das Abgas des Hydrierreaktors 202 wird über Leitung 210 aus dem Hydrierreaktor 202 ausgeführt.

Der Hydrierreaktor 202 umfasst eingebaute Böden 211, welche die Rückvermischung im Hydrierreaktor 202 begrenzen. Die Böden sind vorzugsweise Lochbleche. Dispergiertes Wasserstoffgas wird von den Böden zumindest teilweise zurückgehalten, sodass der Anteil an dispergiertem Gas im flüssigen Reaktionsgemisch im zum Austritt aus dem zweiten Reaktor gelegenen Abschnitt sinkt. Zumindest am Austritt erfolgt die Hydrierung flüssig-einphasig.

Eine das hydrierte Reaktionsprodukt enthaltende Flüssigkeit wird über Leitung 212 aus dem Hydrierreaktor 202 ausgeführt.

Eine einen homogenen Rhodium-Katalysator, der wenigstens einen chiralen Liganden aufweist, umfassende Flüssigkeit wird in den in den Flüssigkeitsstrom 204 eindosiert (nicht dargestellt).

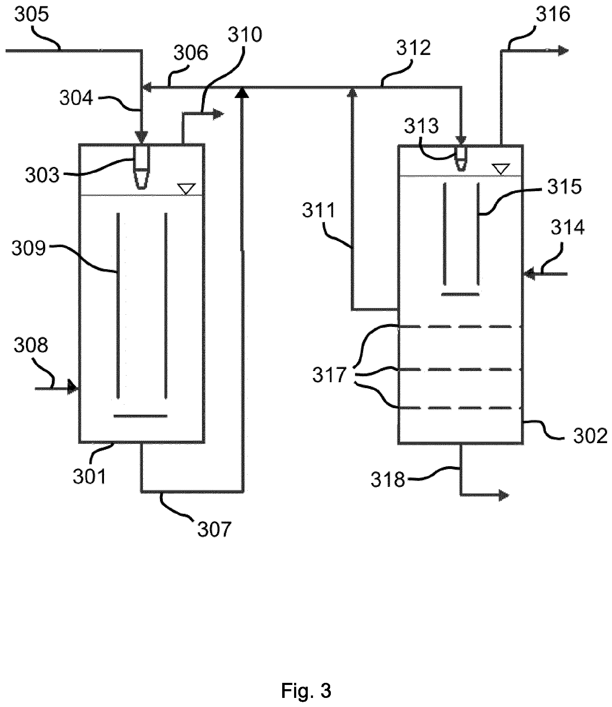

Gemäß Fig. 3 umfasst eine zur Durchführung des erfindungsgemäßen Verfahrens geeignete Anlage einen ersten Hydrierreaktor 301 und einen zweiten Hydrierreaktor 302. Die Hydrierreaktoren 301 und 302 sind injektionsdurchmischt und weisen jeweils einen Umpumpkreislauf auf.

Der Hydrierreaktor 301 umfasst eine Strahldüse 303. Über die Strahldüse 303 wird eine Flüssigkeit aus Leitung 304 dem Hydrierreaktor 301 zugeführt. Der Flüssigkeitsstrom aus Leitung 304 umfasst den Eduktstrom 305 und den Umpumpstrom 306. Der Eduktstrom 305 umfasst eine ein zu hydrierendes Edukt umfassende Flüssigkeit . Der Umpumpstrom 306 ist ein Teilstrom des am Sumpf des Hydrierreaktors 1 ausgeführten Austragsstroms 307. Wasserstoffgas wird dem Hydrierreaktor 301 über Leitung 308 zugeführt. Der Hydrierreaktor 301 umfasst ein Leitrohr 309, wobei die Strahldüse 303 oberhalb des Flüssigkeitsstandes angeordnet und der von der Strahldüse 303 erzeugte Gas/Flüssigkeitsstrahl in das Leitrohr 309 gerichtet ist. Das Abgas des Hydrierreaktors 301 wird über Leitung 310 aus dem Hydrierreaktor 301 ausgeführt.

Ein Teilstrom des Austragsstroms 307 wird mit dem aus der rückvermischten Zone des Hydrierreaktors 302 entnommenen flüssigen Umpumpstrom 311 zu Flüssigkeitsstrom 312 vereint und dem Hydrierreaktor 302 über die Strahldüse 313 zugeführt. Wasserstoffgas wird dem Hydrierreaktor 302 über Leitung 314 zugeführt. Der Hydrierreaktor 302 umfasst ein Leitrohr 315, wobei die Strahldüse 313 oberhalb des Flüssigkeitsstandes angeordnet und der von der Strahldüse 313 erzeugte Gas/Flüssigkeitsstrahl in das Leitrohr 315 gerichtet ist. Das Abgas des Hydrierreaktors 302 wird über Leitung 316 aus dem Hydrierreaktor 302 ausgeführt.

Der Hydrierreaktor 302 umfasst eingebaute Böden 317, welche die Rückvermischung im Hydrierreaktor 302 begrenzen. Die Böden sind vorzugsweise Lochbleche. Dispergiertes Wasserstoffgas wird von den Böden zumindest teilweise zurückgehalten, sodass der Anteil an dispergiertem Gas im flüssigen Reaktionsgemisch im zum Austritt aus dem zweiten Reaktor gelegenen Abschnitt sinkt. Zumindest am Austritt erfolgt die Hydrierung flüssig-einphasig.

Eine das hydrierte Reaktionsprodukt enthaltende Flüssigkeit wird über Leitung 318 aus dem Hydrierreaktor 302 ausgeführt.

Eine einen homogenen Rhodium-Katalysator, der wenigstens einen chiralen Liganden aufweist, umfassende Flüssigkeit wird in den Umpumpkreislauf des Hydrierreaktors 301, beispielsweise in den Flüssigkeitsstrom 304, eindosiert (nicht dargestellt). Der Umpumpkreislauf des Hydrierreaktors 301 und des Hydrierreaktors 302 umfasst einen externen Wärmetauscher (nicht dargestellt), welcher beispielsweise den Austragsstrom 307 und den Umpumpstrom 311 kühlt.

### Beispiel

In einem Strahlschlaufenreaktor mit einem Flüssigkeitsvolumen von 12 m³ wurden 1.500 kg/h Citral und 1.500 kg/h einer Katalysatormischung eingespeist. Bei der Katalysatormischung handelte es sich um eine analog der in US 2018/057437 A1, WO 2006/040096 und WO 2008/132057 A1 beschriebenen Vorschriften hergestellte Mischung, wobei Rh(CO)₂acac, Chiraphos und Tridodecylamin im molaren Verhältnis 1 : 1,4 : 10 in Citronellal mit CO und H₂ umgesetzt wurden. Die Konzentration der Katalysatormischung im Strahlschlaufenreaktor betrug 300 bis 1.000 Gew.-ppm, bezogen auf die Menge an Rhodium im Katalysator. Das Verhältnis von externem Umlauf zu Zulauf betrug 380 : 130. Das Verhältnis von internem Umlauf zum Zulauf betrug 3.000 : 1000.

Der Leistungseintrag betrug 2 kW/m³. Der Druck im Reaktor wurde mittels Einspeisung von Wasserstoffgas (enthaltend 1.000 ppm Kohlenmonoxid) auf 80 bar reguliert. Die Temperatur im Reaktor wurde auf 22 °C reguliert. Der Umsatz des Citrals betrug 88%. Im Austrag des ersten Reaktors lag eine Citral-Konzentration von etwa 7 Gew.-% vor.

Der Reaktoraustrag wurde in einen zweiten Reaktor mit einem Flüssigkeitsvolumen von 9,7 m³ eingespeist. Der Druck im zweiten Reaktor wurde mittels Einspeisung von Wasserstoffgas (enthaltend 1.000 ppm Kohlenmonoxid) auf 80 bar reguliert. Die Temperatur im Reaktor wurde auf 22 °C reguliert. Der Gesamtumsatz nach dem zweiten Reaktor lag zwischen 93 und 99,9 %.

Es zeigte sich, dass die Reaktionsgeschwindigkeit im ersten Reaktor bei höheren Citral-Konzentrationen abnahm. Es erwies sich somit als vorteilhaft, den ersten Reaktor bei niedrigen Citral-Konzentrationen zu betreiben und die Reaktion im zweiten Reaktor zu vervollständigen.

## Patentansprüche

1. Verfahren zur kontinuierlichen Herstellung einer optisch aktiven Carbonylverbindung durch asymmetrische Hydrierung einer prochiralen α,β-ungesättigten Carbonylverbindung mit Wasserstoff in Gegenwart eines homogenen Rhodium-Katalysators, der wenigstens einen chiralen Liganden aufweist, wobei man
ein die prochirale α,β-ungesättigte Carbonylverbindung enthaltendes flüssiges Reaktionsgemisch in einem ersten, rückvermischten Reaktor einer gas/flüssigzweiphasigen Hydrierung unterzieht, und
das flüssige Reaktionsgemisch dann in einem zweiten Reaktor weiter hydriert,
wobei man in dem ersten Reaktor eine Konzentration der prochiralen α,β-ungesättigten Carbonylverbindung von 3 bis 20 Gew.-% einstellt.

2. Verfahren nach Anspruch 1, wobei man in einem zum Eintritt in den zweiten Reaktor gelegenen Abschnitt des zweiten Reaktors Wasserstoffgas in dem flüssigen Reaktionsgemisch dispergiert.

3. Verfahren nach Anspruch 1 oder 2, wobei man in dem ersten Reaktor eine Konzentration der prochiralen α,β-ungesättigten Carbonylverbindung einstellt, bei der die Reaktionsgeschwindigkeit wenigstens das 0,8-fache von Vₘₐₓ beträgt und Vₘₐₓ das Maximum der Reaktionsgeschwindigkeit bei Auftragung der Reaktionsgeschwindigkeit gegen die Konzentration der prochiralen α,β-ungesättigten Carbonylverbindung ist.

4. Verfahren nach einem der vorhergehenden Ansprüche, wobei man in dem zweiten Reaktor das flüssige Reaktionsgemisch bis zu einer Konzentration der prochiralen α,β-ungesättigten Carbonylverbindung von weniger als 5 Gew.-% umsetzt.

5. Verfahren nach einem der vorhergehenden Ansprüche, wobei das Verhältnis des Reaktionsvolumens des ersten Reaktors zum Reaktionsvolumen des zweiten Reaktors 1:1 bis 1:5 beträgt.

6. Verfahren nach einem der vorhergehenden Ansprüche, wobei der erste Reaktor durch eine Kesselzahl N im Bereich von 1 bis 3 gekennzeichnet ist.

7. Verfahren nach einem der vorhergehenden Ansprüche, wobei der volumenspezifische Leistungseintrag in den ersten Reaktor 0,5 bis 5 kW/m³ beträgt.

8. Verfahren nach einem der vorhergehenden Ansprüche, wobei der erste Reaktor als Schlaufenreaktor ausgebildet ist.

9. Verfahren nach einem der vorhergehenden Ansprüche, wobei man eine Rückvermischung im zweiten Reaktor begrenzt und wobei man zumindest in einem zum Austritt aus dem zweiten Reaktor gelegenen Abschnitt des zweiten Reaktors die Hydrierung flüssig-einphasig durchführt.

10. Verfahren nach Anspruch 9, wobei die Rückvermischung in dem zweiten Reaktor durch Einbauten begrenzt ist.

11. Verfahren nach einem der vorhergehenden Ansprüche, wobei der zweite Reaktor durch eine Kesselzahl N von mehr als 4 gekennzeichnet ist.

12. Verfahren nach einem der vorhergehenden Ansprüche, wobei die prochirale α,β-ungesättigte Carbonylverbindung ausgewählt ist unter Verbindungen der allgemeinen Formel (I) 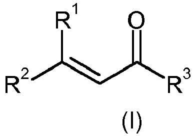 worin
R¹, R² voneinander verschieden sind und jeweils für einen unverzweigten, verzweigten oder cyclischen Kohlenwasserstoffrest mit 1 bis 25 Kohlenstoffatomen stehen, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen,
R³ für Wasserstoff oder für einen unverzweigten, verzweigten oder cyclischen Kohlenwasserstoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen,
oder
R³ gemeinsam mit einem der Reste R¹ oder R² auch eine 3 bis 25-gliedrige Alkylengruppe bedeuten kann worin 1, 2, 3 oder 4 nicht benachbarte CH₂-Gruppen durch O oder N-R^{5c} ersetzt sein können, wobei die Alkylengruppe gesättigt ist oder eine oder mehrere, nicht konjugierte ethylenische Doppelbindungen aufweist, und wobei die Alkylengruppe unsubstituiert ist oder einen oder mehrere gleiche oder verschiedene Substituenten trägt, die ausgewählt sind unter OR⁴, NR^{5a}R^{5b}, Halogen, C₁-C₄-Alkyl, C₆-C₁₀-Aryl und Hetaryl mit 5 bis 10 Ringatomen, wobei zwei Substituenten auch gemeinsam für eine 2 bis 10-gliedrige Alkylengruppe stehen können, wobei die 2-bis 10-gliedrige Alkylengruppe gesättigt ist oder eine oder mehrere, nicht konjugierte ethylenische Doppelbindungen aufweist, und wobei die 2- bis 10-gliedrige Alkylengruppe unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen;
wobei
R⁴ für Wasserstoff, C₁-C₆-Alkyl, C₆-C₁₀-Aryl, C₆-C₁₄-Aryl-C₁-C₁₀-alkyl, oder C₁-C₁₀-Alkyl-C₆-C₁₄-aryl- steht;
R^{5a}, R^{5b} jeweils unabhängig voneinander Wasserstoff, C₁-C₆-Alkyl, C₆-C₁₀-Aryl, C₆-C₁₄-Aryl-C₁-C₁₀-alkyl oder C₁-C₁₀-Alkyl-C₆-C₁₄-aryl bedeuten oder
R^{5a} und R^{5b} gemeinsam auch eine Alkylenkette mit 2 bis 5 Kohlenstoffatomen, die durch N oder O unterbrochen sein kann, bedeuten können; und
R^{5c} für Wasserstoff, C₁-C₆-Alkyl, C₆-C₁₀-Aryl, C₆-C₁₄-Aryl-C₁-C₁₀-alkyl oder C₁-C₁₀-Alkyl-C₆-C₁₄-ary- steht.

13. Verfahren nach Anspruch 12 zur Herstellung von optisch aktivem Citronellal der Formel (III) 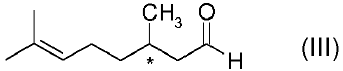 worin * das Asymmetriezentrum bezeichnet;
durch asymmetrische Hydrierung von Geranial der Formel (la-1) oder von Neral der Formel (lb-1) 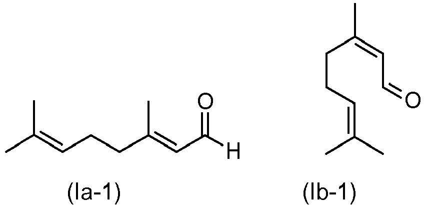
oder einer Neral und Geranial enthaltenden Mischung.

14. Verfahren nach einem der vorhergehenden Ansprüche, wobei die Katalysatorkonzentration 0,001 bis 1 mol-% bezogen auf die Menge an prochiraler α,β-ungesättigter Carbonylverbindung im Reaktionsgemisch, gerechnet als im Katalysator enthaltene Rhodium-Atome, beträgt.

15. Verfahren nach einem der vorhergehenden Ansprüche, wobei der chirale Ligand ein chiraler zweizähniger Bisphosphin-Ligand ist, insbesondere Chiraphos.

16. Verfahren nach einem der vorhergehenden Ansprüche, wobei das Verfahren in Gegenwart einer Verbindung der Formel (II) durchgeführt wird, 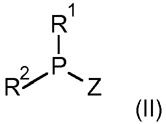 worin Z in Formel (II) für eine Gruppe CHR³R⁴ steht und worin die Variablen R¹, R², R³, R⁴ unabhängig voneinander und insbesondere gemeinsam die folgenden Bedeutungen aufweisen:
R¹, R²: sind gleich oder verschieden und stehen für Phenyl, das unsubstituiert ist oder 1, 2 oder 3 Substituenten trägt, die ausgewählt sind unter Methyl und Methoxy, wobei R¹ und R² jeweils insbesondere für unsubstituiertes Phenyl stehen;
R³ steht für C₁- bis C₄-Alkyl, insbesondere für Methyl;
R⁴ steht für C₁- bis C₄-Alkyl, das eine Gruppe P(=O)R^{4a}R^{4b} trägt und insbesondere eine Gruppe CH₂-P(=O)R^{4a}R^{4b} oder CH(CH₃)-P(=O)R^{4a}R^{4b} bedeutet;
wobei
R^{4a}, R^{4b}: gleich oder verschieden sind und für Phenyl stehen, das unsubstituiert ist oder 1, 2 oder 3 Substituenten trägt, die ausgewählt sind unter Methyl und Methoxy, wobei besonders bevorzugt R^{4a} und R^{4b} jeweils für unsubstituiertes Phenyl stehen.

17. Verfahren zur Herstellung von optisch aktivem Menthol, bei dem man optisch aktives Citronellal der Formel (III) nach einem Verfahren der Ansprüche 13 bis 16 herstellt, das optisch aktive Citronellal der Formel (III) einer Cyclisierung zu optisch aktivem Isopulegol unterzieht und das optisch aktive Isopulegol zu optisch aktivem Menthol hydriert.

## Claims

1. A process for the continuous production of an optically active carbonyl compound by asymmetric hydrogenation of a prochiral α,β-unsaturated carbonyl compound with hydrogen in the presence of a homogeneous rhodium catalyst that has at least one chiral ligand, wherein
a liquid reaction mixture comprising the prochiral α,β-unsaturated carbonyl compound is subjected in a first, backmixed reactor to a gas/liquid two-phase hydrogenation, and
the liquid reaction mixture is then further hydrogenated in a second reactor,
wherein the prochiral α,β-unsaturated carbonyl compound is employed in the first reactor in a concentration from 3% to 20% by weight.

2. The process according to claim 1, wherein hydrogen gas undergoes dispersion in the liquid reaction mixture in a section of the second reactor located at the entrance to the second reactor.

3. The process according to claim 1 or 2, wherein the prochiral α,β-unsaturated carbonyl compound is employed in the first reactor in a concentration at which the reaction rate is at least 0.8 times Vₘₐₓ, Vₘₐₓ being the maximum value for the reaction rate in a plot of the reaction rate against the concentration of the prochiral α,β-unsaturated carbonyl compound.

4. The process according to any of the preceding claims, wherein the liquid reaction mixture undergoes reaction in the second reactor until the concentration of the prochiral α,β-unsaturated carbonyl compound is less than 5% by weight.

5. The process according to any of the preceding claims, wherein the ratio of the reaction volume of the first reactor to the reaction volume of the second reactor is 1:1 to 1:5.

6. The process according to any of the preceding claims, wherein the first reactor is **characterized by** a reactor number N within a range from 1 to 3.

7. The process according to any of the preceding claims, wherein the volume-specific power input into the first reactor is 0.5 to 5 kW/m³.

8. The process according to any of the preceding claims, wherein the first reactor is configured as a loop reactor.

9. The process according to any of the preceding claims, wherein backmixing in the second reactor is limited and wherein, at least in a section of the second reactor located at the exit from the second reactor, the hydrogenation is carried out in liquid single-phase.

10. The process according to claim 9, wherein backmixing in the second reactor is limited by internals.

11. The process according to any of the preceding claims, wherein the second reactor is **characterized by** a reactor number N of more than 4.

12. The process according to any of the preceding claims, wherein the prochiral α,β-unsaturated carbonyl compound is selected from compounds of the general formula (I) 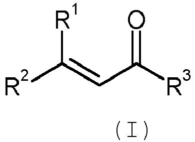 where
R¹, R² are different from one another and are each an unbranched, branched or cyclic hydrocarbon radical having 1 to 25 carbon atoms that is saturated or has one or more unconjugated ethylenic double bonds and that is unsubstituted or bears one or more identical or different substituents selected from OR⁴, NR^{5a}R^{5b}, halogen, C₆-C₁₀ aryl and heteroaryl having 5 to 10 ring atoms,
R³ is hydrogen or an unbranched, branched or cyclic hydrocarbon radical having 1 to 25 carbon atoms that is saturated or has one or more unconjugated ethylenic double bonds and that is unsubstituted or bears one or more identical or different substituents selected from OR⁴, NR^{5a}R^{5b}, halogen, C₆-C₁₀ aryl and heteroaryl having 5 to 10 ring atoms,
or
R³ jointly with either of the radicals R¹ or R² may also represent a 3- to 25-membered alkylene group wherein 1, 2, 3 or 4 nonadjacent CH₂-groups may be replaced by O or N-R^{5c}, wherein the alkylene group is saturated or has one or more unconjugated ethylenic double bonds and wherein the alkylene group is unsubstituted or bears one or more identical or different substituents selected from OR⁴, NR^{5a}R^{5b}, halogen, C₁-C₄-alkyl, C₆-C₁₀-aryl and heteroaryl having 5 to 10 ring atoms, wherein two substituents may also jointly represent a 2- to 10-membered alkylene group, wherein the 2- to 10-membered alkylene group is saturated or has one or more unconjugated ethylenic double bonds and wherein the 2- to 10-membered alkylene group is unsubstituted or bears one or more identical or different substituents selected from OR⁴, NR^{5a}R^{5b}, halogen, C₆-C₁₀-aryl and heteroaryl having 5 to 10 ring atoms;
where
R⁴ is hydrogen, C₁-C₆ alkyl, C₆-C₁₀ aryl, C₆-C₄ aryl-C₁-C₁₀ alkyl, or C₁-C₁₀ alkyl-C₆-C₁₄ aryl;
R^{5a}, R^{5b} are each independently hydrogen, C₁-C₆ alkyl, C₆-C₁₀ aryl, C₆-C₁₄ aryl-C₁-C₁₀ alkyl or C₁-C₁₀ alkyl-C₆-C₁₄ aryl or
R^{5a} and R^{5b} may also jointly represent an alkylene chain having 2 to 5 carbon atoms, which may be interrupted by N or O; and
R^{5c} is hydrogen, C₁-C₆ alkyl, C₆-C₁₀ aryl, C₆-C₄ aryl-C₁-C₁₀ alkyl or C₁-C₁₀ alkyl-C₆-C₁₄ aryl.

13. The process according to claim 12 for producing optically active citronellal of the formula (III) 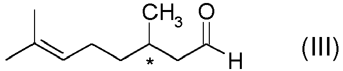 where * denotes the asymmetric center;
by asymmetric hydrogenation of geranial of the formula (Ia-1) or of neral of the formula (Ib-1) 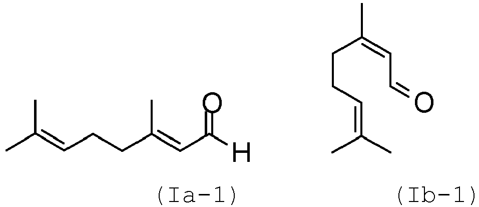
or of a mixture comprising neral and geranial.

14. The process according to any of the preceding claims, wherein the catalyst concentration is 0.001 to 1 mol% based on the amount of prochiral α,β-unsaturated carbonyl compound in the reaction mixture, calculated as rhodium atoms present in the catalyst.

15. The process according to any of the preceding claims, wherein the chiral ligand is a chiral bidentate bisphosphine ligand, more particularly chiraphos.

16. The process according to any of the preceding claims, wherein the process is executed in the presence of a compound of the formula (II), 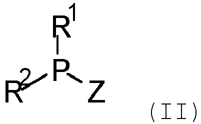 where Z in formula (II) is a CHR³R⁴ group and where the variables R¹, R², R³, R⁴ are independently, and in particular jointly, as follows:
R¹, R²: are identical or different and are phenyl that is unsubstituted or bears 1, 2 or 3 substituents selected from methyl and methoxy, where R¹ and R² are each in particular unsubstituted phenyl;
R³ is C₁ to C₄ alkyl, in particular methyl;
R⁴ is C₁ to C₄ alkyl bearing a P(=O)R^{4a}R^{4b} group and in particular a CH₂-P(=O)R^{4a}R^{4b} or CH(CH₃)-P(=O)R^{4a}R^{4b} group;
where
R^{4a}, R⁴b: are identical or different and are phenyl that is unsubstituted or bears 1, 2 or 3 substituents selected from methyl and methoxy, where R^{4a} and R^{4b} are particularly preferably each unsubstituted phenyl.

17. A process for producing optically active menthol in which optically active citronellal of the formula (III) is produced in a process according to claims 13 to 16, the optically active citronellal of the formula (III) is subjected to a cyclization to afford optically active isopulegol and the optically active isopulegol is hydrogenated to afford optically active menthol.

## Revendications

1. Procédé pour la préparation continue d'un composé carbonyle optiquement actif par hydrogénation asymétrique d'un composé carbonyle prochiral α,β-insaturé avec de l'hydrogène en présence d'un catalyseur homogène à base de rhodium, qui présente au moins un ligand chiral, un mélange réactionnel liquide contenant le composé carbonyle prochiral α,β-insaturé étant soumis à une hydrogénation en deux phases gaz/liquide dans un premier réacteur à mélange en retour et le mélange réactionnel liquide étant ensuite hydrogéné dans un deuxième réacteur, la concentration en composé carbonyle prochiral α,β-insaturé dans le premier réacteur étant réglée à 3 jusqu'à 20% en poids.

2. Procédé selon la revendication 1, de l'hydrogène gazeux étant dispersé dans le mélange réactionnel liquide dans une section du deuxième réacteur se trouvant à l'entrée du deuxième réacteur.

3. Procédé selon la revendication 1 ou 2, la concentration en composé carbonyle prochiral α,β-insaturé dans le premier réacteur étant réglée de manière telle que la vitesse de réaction représente au moins 0,8 fois Vₘₐₓ et Vₘₐₓ étant le maximum de la vitesse de réaction lorsqu'on reporte la vitesse de réaction par rapport à la concentration en composé carbonyle prochiral α,β-insaturé.

4. Procédé selon l'une quelconque des revendications précédentes, le mélange réactionnel liquide étant transformé dans le deuxième réacteur jusqu'à une concentration en composé carbonyle prochiral α,β-insaturé inférieure à 5% en poids.

5. Procédé selon l'une quelconque des revendications précédentes, le rapport du volume de réaction du premier réacteur au volume de réaction de deuxième réacteur étant de 1:1 à 1:5.

6. Procédé selon l'une quelconque des revendications précédentes, le premier réacteur étant **caractérisé par** un indice de cuve N dans la plage de 1 à 3.

7. Procédé selon l'une quelconque des revendications précédentes, l'introduction de puissance spécifique au volume dans le premier réacteur étant de 0,5 à 5 kW/m³.

8. Procédé selon l'une quelconque des revendications précédentes, le premier réacteur étant conçu sous forme de réacteur à boucle à circulation interne.

9. Procédé selon l'une quelconque des revendications précédentes, le mélange en retour dans le deuxième réacteur étant limité et l'hydrogénation au moins dans une section du deuxième réacteur située à la sortie de deuxième réacteur étant réalisée dans une seule phase liquide.

10. Procédé selon la revendication 9, le mélange en retour dans le deuxième réacteur étant limité par des encastrements.

11. Procédé selon l'une quelconque des revendications précédentes, le deuxième réacteur étant **caractérisé par** un indice de cuve N supérieur à 4.

12. Procédé selon l'une quelconque des revendications précédentes, le composé carbonyle prochiral α,β-insaturé étant choisi parmi les composés de formule générale (I) 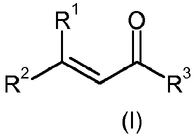 dans laquelle
R¹, R² sont différents l'un de l'autre et représentent à chaque fois un radical hydrocarbonée non ramifié, ramifié ou cyclique comprenant 1 à 25 atomes de carbone, qui est saturé ou qui présente une ou plusieurs doubles liaisons éthyléniques non conjuguées et qui est non substitué ou qui porte un ou plusieurs substituants identiques ou différents qui sont choisis parmi OR⁴, NR^{5a}R^{5b}, halogène, C₆-C₁₀-aryle et hétéroaryle comprenant 5 à 10 atomes de cycle,
R³ représente hydrogène ou un radical hydrocarboné non ramifié, ramifié ou cyclique comprenant 1 à 25 atomes de carbone, qui est saturé ou qui présente une ou plusieurs doubles liaisons éthyléniques non conjuguées et qui est non substitué ou qui porte un ou plusieurs substituants identiques ou différents qui sont choisis parmi OR⁴, NR^{5a}R^{5b}, halogène, C₆-C₁₀-aryle et hétéroaryle comprenant 5 à 10 atomes de cycle ou
R³ peut également signifier, conjointement avec l'un des radicaux R¹ ou R², un groupe alkylène de 3 à 25 chaînons dans lequel 1, 2, 3 ou 4 groupes CH₂ non adjacents pouvant être remplacés par O ou N-R^{5c}, le groupe alkylène étant saturé ou présentant une ou plusieurs doubles liaisons éthyléniques non conjuguées et le groupe alkylène étant non substitué ou portant un ou plusieurs substituants identiques ou différents, qui sont choisis parmi OR⁴, NR^{5a}R^{5b}, halogène, C₁-C₄-alkyle, C₆-C₁₀-aryle et hétéroaryle comprenant 5 à 10 atomes de cycle, deux substituants pouvant également représenter conjointement un groupe alkylène de 2 à 10 chaînons, le groupe alkylène de 2 à 10 chaînons étant saturé ou présentant une ou plusieurs doubles liaisons éthyléniques non conjuguées et le groupe alkylène de 2 à 10 chaînons étant non substitué ou portant un ou plusieurs substituants identiques ou différents qui sont choisis parmi OR⁴, NR^{5a}R^{5b}, halogène, C₆-C₁₀-aryle et hétéroaryle comprenant 5 à 10 atomes de cycle ;
où
R⁴ représente hydrogène, C₁-C₆-alkyle, C₆-C₁₀-aryle, C₆-C₁₄-aryl-C₁-C₁₀-alkyle ou C₁-C₁₀-alkyl-C₆-C₁₄-aryle ;
R^{5a}, R^{5b} signifient, à chaque fois indépendamment l'un de l'autre, hydrogène, C₁-C₆-alkyle, C₆-C₁₀-aryle, C₆-C₁₄-aryl-C₁-C₁₀-alkyle ou C₁-C₁₀-alkyl-C₆-C₁₄-aryle ou
R^{5a} et R^{5b} peuvent également signifier, conjointement, une chaîne alkylène comprenant 2 à 5 atomes de carbone, qui peut être interrompue par N ou O ; et
R^{5c} représente hydrogène, C₁-C₆-alkyle, C₆-C₁₀-aryle, C₆-C₁₄-aryl-C₁-C₁₀-alkyle ou C₁-C₁₀-alkyl-C₆-C₁₄-aryle.

13. Procédé selon la revendication 12 pour la préparation de citronellal optiquement actif de formule (III) 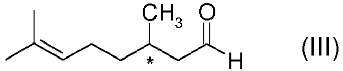 dans laquelle * désigne le centre d'asymétrie ;
par hydrogénation asymétrique de géranial de formule (Ia-1) ou de néral de formule (Ib-1) 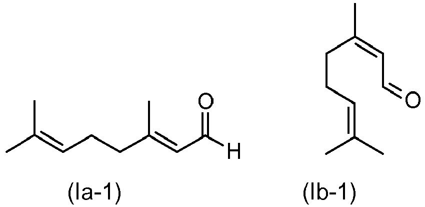
ou d'un mélange contenant du néral et du géranial.

14. Procédé selon l'une quelconque des revendications précédentes, la concentration en catalyseur étant de 0,001 à 1% en mole par rapport à la quantité de composé carbonyle prochiral α,β-insaturé dans le mélange réactionnel, exprimé en atomes de rhodium contenus dans le catalyseur.

15. Procédé selon l'une quelconque des revendications précédentes, le ligand chiral étant un ligand chiral bidentate de diphosphine, en particulier le Chiraphos.

16. Procédé selon l'une quelconque des revendications précédentes, le procédé étant réalisé en présence d'un composé de formule (II) 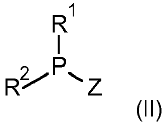 Z dans la formule (II) représentant un groupe CHR³R⁴ et les variables R¹, R², R³, R⁴ présentant, indépendamment les unes des autres et en particulier conjointement, les significations suivantes :
R¹, R² : sont identiques ou différents et représentent phényle qui est non substitué ou qui porte 1, 2 ou 3 substituants qui sont choisis parmi méthyle et méthoxy, R¹ et R² représentant à chaque fois en particulier phényle non substitué ;
R³ représente C₁-C₄-alkyle, en particulier méthyle ;
R⁴ représente C₁-C₄-alkyle, qui porte un groupe P (=O)R^{4a}R^{4b} et signifie en particulier un CH₂-P (=O)R^{4a}R^{4b} ou CH (CH₃) -P (=O)R^{4a}R^{4b} ;
où
R^{4a}, R^{4b} : sont identiques ou différents et représentent phényle qui est non substitué ou qui porte 1, 2 ou 3 substituants qui sont choisis parmi méthyle et méthoxy, R^{4a} et R^{4b} représentant à chaque fois de manière particulièrement préférée phényle non substitué.

17. Procédé pour la préparation de menthol optiquement actif, dans lequel on prépare du citronellal optiquement actif de formule (III) selon un procédé des revendications 13 à 16, on soumet le citronellal optiquement actif de formule (III) à une cyclisation en isopulégol optiquement actif et on hydrogène l'isopulégol optiquement actif en menthol optiquement actif.
